# EUROPÄISCHE PATENTANMELDUNG

(11) **EP 2 298 371 A1**
(43) Veröffentlichungstag der Anmeldung: **23.03.2011**
(21) Anmeldenummer: 09075439.1
(22) Anmeldetag: 22.09.2009
(51) Int. Cl.: A61M 1/10, A61M 1/12, F04D 29/24

(54) **Funktionselement, insbesondere Fluidpumpe, mit einem Gehäuse und einem Förderelement**

(71) Anmelder: ECP Entwicklungsgesellschaft mbH, 12247 Berlin (DE)
(72) Erfinder: Scheckel, Mario, 13187 Berlin (DE)
(74) Vertreter: Pfenning, Meinig & Partner GbR

(57) **Zusammenfassung**

Die Erfindung bezieht sich auf ein Funktionselement (1), insbesondere eine Fluidpumpe, mit einem einen Fluidraum (13) begrenzenden Gehäuse (5) und mit einem in dem Fluidraum angeordneten Interaktionselement (8), insbesondere Förderelement für das Fluid, wobei das Gehäuse (5) bezüglich seiner Form und/oder Größe zwischen wenigstens einem ersten, expandierten und einem zweiten, komprimierten Zustand veränderbar ist. Die Aufgabe, ein entsprechendes Gehäuse ausreichend zu stabilisieren, wird gemäß der Erfindung dadurch gelöst, dass das Gehäuse wenigstens eine mit einem Fluiddruck beaufschlagbare, von dem Fluidraum verschiedene Stabilisierungskammer (19,20,25,26,31) aufweist, wobei einem ersten Fluiddruck in der Stabilisierungskammer der erste Zustand des Gehäuses und einem zweiten Fluiddruck der zweite Zustand des Gehäuses zugeordnet ist.

## Beschreibung

Die vorliegende Erfindung liegt auf dem Gebiet des Maschinenbaus, insbesondere der Mikromechanik, und ist bei Funktionselementen einsetzbar, die zur Förderung bzw. Beeinflussung von Fluiden eingesetzt werden.

Mit besonderem Vorteil ist die Erfindung in der Medizintechnik verwendbar, wo besonders bei der invasiven Medizin auf Körperflüssigkeiten, beispielsweise Blut, eingewirkt wird. Zu diesem Zweck sind mikromechanische Funktionselemente, beispielsweise Pumpen, bekannt, die derart klein gebaut werden, dass sie durch ein Blutgefäß befördert werden können. Derartige Pumpen können innerhalb eines Blutgefäßes selbst oder in einer Herzkammer betrieben werden.

Um einen besonders effizienten und effektiven Betrieb zu ermöglichen, ist es bekannt, derartige Funktionselemente so zu gestalten, dass sie einen komprimierten Zustand aufweisen, in dem sie durch eine Blutbahn bewegt werden können, sowie einen expandierten oder dilatierten Zustand, den sie beispielsweise nach dem Einbringen in eine Herzkammer oder einen anderen Körperhohlraum einnehmen können. In diesem expandierten Zustand kann dann beispielsweise eine Pumpe einen Rotor und ein Gehäuse aufweisen, die durch ihre Größe eine ausreichende Pumpleistung aufbringen und dennoch im komprimierten Zustand in den Körper eingeführt und aus diesem wieder entfernt werden können.

Zur Komprimierung bzw. Expandierung solcher Funktionselemente sind verschiedene Techniken bekannt. Beispielsweise werden sogenannte Formgedächtnismaterialien in Form von Legierungen verwendet, von denen eine unter dem Markennamen Nitinol bekannt ist, wobei die entsprechenden Bauelemente in der Regel temperaturabhängig verschiedene geometrische Formen annehmen. Die Konstruktionsart kann dann so gestaltet sein, dass in einem ersten Zustand eines Nitinolgerüsts eine erste Baugröße eingenommen wird, während in einem zweiten Zustand bei einer anderen Temperatur eine zweite Größe erreicht wird. Auf diese Weise sind beispielsweise bei Pumpen sowohl die Gehäuse als auch die Rotoren im Prinzip komprimierbar und expandierbar.

Es sind jedoch auch Pumpen ohne Rotoren bekannt, die mittels Volumenänderungen durch Verdrängung ein Fluid transportieren. Eine derartige Pumpe ist beispielsweise aus der DE 10 2007 012 817 A1 bekannt. Dort ist eine Verwendung einer solchen torusförmigen Pumpe zur Herzunterstützung bekannt, wobei die Pumpe in einem Blutgefäß eingesetzt ist. Das Gehäuse weist eine stabile Außenform auf, ist jedoch zusammenfaltbar. Die ganze Pumpe ist als Doppelkammerhohlkörper ausgeführt, wobei ein innerhalb des Gehäuses veränderbarer Ballon die Volumenänderung eines Pumpvolumens bewirkt und damit Flüssigkeit ansaugt und auspresst. Zur Art des Gehäuses und dazu, in welcher Art dieses Gehäuse komprimierbar ist, ist in dem Dokument nichts ausgeführt.

Aus der Offenlegungsschrift 4124299 A1 ist ein Pumpe bekannt, die ein mit einem Fluid füllbares und dadurch versteifbares Gehäuse sowie ein darin aufpumpbares Förderelement aufweist, so dass im Gehäuseinnenraum Fluid abwechselnd eingesaugt und verdrängt werden kann.

Ähnliche Pumpen sind aus der US 5820542 und der US 5827171 bekannt.

Vor dem Hintergrund des Standes der Technik liegt der vorliegenden Erfindung die Aufgabe zugrunde, bei einem Funktionselement mit einem Fluidraum und einem diesen begrenzenden Gehäuse sowie einem Interaktionselement, das in dem Fluidraum angeordnet ist, das Gehäuse so auszuführen, dass es bezüglich des Radius komprimierbar und andererseits expandierbar ist und im expandierten Zustand eine möglichst hohe Stabilität aufweist.

Die Aufgabe wird mit den Merkmalen der Erfindung gemäß Patentanspruch 1 gelöst.

Bei einem Funktionselement der eingangs genannten Art ist das Gehäuse bezüglich seiner Form und/oder Größe zwischen wenigstens einem ersten, expandierten und einem zweiten, komprimierten Zustand veränderbar, dadurch, dass es wenigstens eine mit einem Fluiddruck beaufschlagbare, von dem Fluidraum verschiedene Stabilisierungskammer aufweist, wobei einem ersten Fluiddruck in der Stabilisierungskammer der erste Zustand des Gehäuses und einem zweiten Fluiddruck der zweite Zustand des Gehäuses zugeordnet ist.

Die unterschiedliche Füllung bzw. Druckbeaufschlagung der Stabilisierungskammern bewirkt unterschiedliche Materialspannungen in den elastischen Wänden des Gehäuses. Beispielsweise kann die Stabilisierungskammer oder können mehrere Stabilisierungskammern in die Gehäusewände eingelassen sein, und dies wird typischerweise dazu führen, dass bei geringem Druck in den Stabilisierungskammern das Gehäuse sich elastisch zusammenzieht und erschlafft, die Gehäusewände beweglich werden und der Gehäusedurchmesser sowohl bezüglich des Außendurchmessers als auch bezüglich des Innendurchmessers sich verringert. Zu diesem Zweck ist zumindest die das Förderelement direkt umgebende Innen- und/oder Außenwand des Gehäuses, die ihrerseits gegebenenfalls von Stabilisierungskanmmern oder einer Stabilisierungskammer umgeben ist, elastisch ausgebildet. Bei einem erhöhten Druck straffen sich die Stabilisierungskammern, und damit werden die Wände zunächst aufgeweitet und versteift, so dass das Gehäuse expandiert. Die Zustände können jedoch auch so gewählt werden, dass die Wände des Gehäuses Vorspannungen aufweisen, die dazu führen, dass bei einer hohen Druckbeaufschlagung der Stabilisierungskammer(n) ein Zusammenpressen des Gehäuses erfolgt, während bei geringem Druck in den Stabilisierungskammern eine Expansion des Gehäuses durch die Eigenspannung der Gehäusewände erfolgt.

Dabei kann vorteilhaft vorgesehen sein, dass im zweiten Zustand der Durchmesser des Gehäuses durch die Eigenelastizität soweit reduziert ist. Dass das Interaktionselement in seinem expandierten Zustand radial zusammengedrückt wird.

Damit erleichtert die elastische Kompressionswirkung des Gehäuses die Kompression des Interaktionselementes, beispielsweise eines Fluidförderelementes. Dies kann beispielsweise die Form eines Pumpenrotors aufweisen.

Als Fluid kann dabei entweder ein Gas oder eine Flüssigkeit, insbesondere eine biokompatible Flüssigkeit wie Salzlösung, gewählt werden. Vorteilhaft kann das Gehäuse wenigstens abschnittsweise die Form eines Hohlzylinders, eines Torus oder eines Hohlrotationsellipsoids aufweisen. Eine derartige Form führt grundsätzlich zu einer guten Verwendbarkeit des Gehäuses in einem Körper, da diese Formen grundsätzlich komfortabel durch ein körpereigenes Gefäß zu bewegen sind. Eine rotationssymmetrische Form bietet sich insbesondere dann an, wenn das Gehäuse für eine Fluidpumpe verwendet wird. Üblicherweise wird das Gehäuse an den Enden abgeschlossen sein, entweder durch flache Stirnseiten oder durch konische oder auf andere Weise rotationssymmetrische Abschlussflächen mit entsprechenden Einström/Ausströmöffnungen.

Die Stabilisierungskammer kann besonders effizient in der Form eines im ersten Gehäusezustand strangförmigen Hohlraums vorgesehen sein. In diesem Fall bildet die Stabilisierungskammer bei hoher Druckbeaufschlagung gleichsam einen Stabilisierungssteg in der Gehäusewand. Es ist damit eine relativ geringe Menge Fluids notwendig, um die Stabilisierungskammer(n) zu füllen und das Gehäuse zu stabilisieren.

Es können vorteilhaft eine Mehrzahl, insbesondere drei oder mehr Stabilisierungskammern in Form von strangförmigen Hohlkammern vorgesehen sein. Diese können symmetrisch in dem Gehäuse verteilt sein.

Es kann auch vorteilhaft vorgesehen sein, dass wenigstens ein strangförmiger Hohlraum (betrachtet im expandierten Zustand des Gehäuses) in Umfangsrichtung des Hohlzylinders, Torus oder Hohlrotationsellipsoids umlaufend ausgebildet ist. In diesem Fall ergibt sich eine besonders effiziente Stabilisierungsstruktur für das Gehäuse, die bei Druckbeaufschlagung der Stabilisierungskammer besonders einer Kompression des Gehäuses entgegenwirkt. Dies ist besonders dann vorteilhaft, wenn das Funktionselement als Fluidpumpe ausgeführt ist und innerhalb des Gehäuses zumindest zeitweise ein Saugdruck erzeugt wird, um Körperflüssigkeit, insbesondere Blut, anzusaugen. In diesem Moment ist das Gehäuse empfindlich gegenüber der Tendenz zusammenzufallen und muss besonders gegenüber dieser Kompression stabilisiert werden. Es kann als Stabilisierungskammer auch ein torus- oder hohlzylinderförmiger Körper vorgesehen sein.

Alternativ oder zusätzlich zu einer in Umfangsrichtung verlaufenden strangförmigen Hohlkammer können eine oder mehrere strangförmige Hohlkammern parallel zur Längsachse des Hohlzylinders, Torus oder Hohlrotationsellipsoids in dessen Wandung verlaufen. Auf diese Weise kann ein Stabilisierungsgitter gebildet werden, dass bei geringem Gesamtvolumen der Stabilisierungskammern eine gute Stabilisierung und ein gutes, d. h. ein großes Verhältnis, zwischen dem Radius des Gehäuses im expandierten Zustand und dem Radius im komprimierten Zustand zulässt.

Es kann auch vorteilhaft vorgesehen sein, dass die Stabilisierungskammer den Raum der Gehäusewand im Wesentlichen ausfüllt. Dadurch vereinfacht sich die Struktur des Gehäuses, und dieses kann durch Druckbeaufschlagung schnell und komplikationslos gefüllt bzw. auch wieder geleert werden. Das Gehäuse kann dann beispielsweise die Form eines hohlwandigen Ballons aufweisen.

Zur Stabilisierung kann in vielen Fällen vorgesehen sein, dass die entsprechenden Hohlräume von Stegen des Gehäusematerials durchsetzt sind. Auf diese Weise wird eine Stabilisierung des Gehäuses auch gegenüber einer Relativbewegung von Innen- und Außenwänden erreicht.

Das Funktionselement weist insbesondere dann, wenn es als Fluidpumpe ausgeführt ist, an seinem Gehäuse eine Einströmöffnung und eine Ausströmöffnung auf, die jeweils mit einem Ventil, insbesondere einem Einwegventil, versehen sein können. Auf diese Weise kann sichergestellt werden, dass die Funktion bei Verwendung als pulsierende Pumpe mit einer Ansaugphase und einer Auspressphase optimiert ist. Durch die Einströmöffnung wird dann Körperflüssigkeit eingesaugt, während diese durch die Ausströmöffnung in der Auspressphase ausgeleitet wird.

Grundsätzlich kann ein derartiges Gehäuse auch mit einem Förderelement versehen sein, das als Rotor mit wenigstens einem Schaufelblatt für das Fluid ausgeführt ist. Dabei kann vorgesehen sein, dass das Förderelement durch Anklappen, Zusammenfalten oder Zusammenfallen der Förderschaufeln radial komprimierbar ist.

Dabei kann das/ können die Schaufelblatt/Schaufelblätter elastisch radial zur Rotorachse hin biegbar sein.

Es kann aber auch der Rotor wenigstens teilweise aus einem volumenkompressiblen Material, insbesondere einem Schaumstoff, bestehen.

Der Rotor kann zusätzlich oder alternativ komprimierbare Hohlräume aufweisen.

Dabei kann der Rotor auch nabenlos gestaltet sein, indem das Schaufelblatt selbsttragend als Förderfläche stabilisiert ist und das Drehmoment überträgt.

Im Zusammenhang mit der vorliegenden Erfindung liegt der Vorteil eines komprimierbaren Rotors darin, dass dieser schon durch das Kontrahieren des Gehäuses wenigstens teilweise komprimiert wird, so dass keine hohen zusätzlichen Kräfte aufzubringen sind, wenn die Fluidpumpe durch ein Blutgefäß in einem Körper bewegt werden muss.

Ein weiterer Vorteil der elastischen Kontraktion des Gehäuses liegt darin, dass die Kräfte auf den Rotor regelmäßig radial nach innen wirken. Fällt das Gehäuse lediglich erschlafft zusammen, so ist der Effekt auf den Rotor nicht reproduzierbar.

Vorteilhaft kann es auch vorgesehen sein, dass das Förderelement als fluidfüllbarer und damit bezüglich seines Volumens veränderbarer Hohlkörper/Ballon ausgeführt ist. Damit wird erreicht, dass mit Druckbeaufschlagung des Hohlkörpers eine Verdrängung im Innenraum/Fluidraum des Gehäuses stattfindet, so dass das dort befindliche Fluid ausgepresst wird. Wird die Druckbeaufschlagung des Hohlkörpers reduziert, so wird dieser durch die Elastizität seiner Wände komprimiert und gibt zusätzliches Volumen in dem Gehäuse frei, so dass Fluid von außen in den Fluidraum des Gehäuses eingesaugt wird.

Es ergibt sich somit eine pulsierende Pumpe zur Förderung des Fluids. Vorteilhaft kann dabei das Förderelement in gefüllter Form den Fluidraum des Gehäuses im Wesentlichen ausfüllen. Damit ist der Hub der Pumpe beim Einsaugen und Auspressen von Fluid optimiert.

Vorteilhaft kann das Förderelement mit demselben Fluid gefüllt sein wie die Stabilisierungskammer(n). Da sowohl die Stabilisierungskammer(n) als auch das Förderelement nach dem Einbringen des Funktionselements in den Körper druckbeaufschlagt werden müssen, ergibt sich eine starke Vereinfachung, wenn dabei dasselbe Fluid verwendet werden kann. Üblicherweise wird ein biokompatibles Fluid, beispielsweise eine Salzlösung, verwendet. Es ist jedoch auch die Verwendung eines Gases, beispielsweise eines bioinerten Gases wie Helium denkbar.

Weiter vereinfacht wird die Handhabung, indem das Förderelement und die Stabilisierungskammern des Gehäuses mit derselben Fluiddruckquelle verbindbar sind.

Die Aufgabe der Erfindung, ein Funktionselement, insbesondere eine Fluidpumpe mit einem komprimierbaren Gehäuse herzustellen, wird auch durch das folgende Funktionselement gelöst. Hierbei handelt es sich um ein Funktionselement, insbesondere eine Fluidpumpe, mit einem einen Fluidraum begrenzenden Gehäuse und mit einem in dem Fluidraum angeordneten Interaktionselement, insbesondere Förderelement für das Fluid, wobei sowohl das Gehäuse als auch das Interaktionselement bezüglich seiner Form und/oder Größe zwischen wenigstens einem ersten, expandierten und einem zweiten, komprimierten Zustand veränderbar ist, wobei die mittlere Dichteänderung des Gehäusematerials zwischen dem ersten und zweiten Zustand mindestens 10 % beträgt.

Hierbei ist die mittlere Dichteänderung des Gehäusematerials bei gleich bleibender Temperatur gemeint, beispielsweise bei 36°C. Die lokale Dichteänderung des Gehäuses kann stark variieren, entscheidend ist die mittlere Dichteänderung des Gehäusematerials, wobei dieses Gehäusematerial durchaus nicht homogen sein muss, sondern hier auch beispielsweise metallische Teile Bestandteil sein können, die dann eine entsprechend geringere Dichteänderung aufweisen würden.

Bevorzugt sind hier reversible oder auch irreversible Materialien vorzusehen, die aufgrund osmotischer Funktionsweise im dekomprimierten Zustand ein größeres Volumen / geringere Dichte aufweisen, bzw. Schaumstoffe, die im dekomprimierten Zustand eine geringere Dichte aufweisen. Diese Schaumstoffe können offen- oder geschlossenporig sein.

Das Gehäuse zeichnet sich vorzugsweise durch eine Materialmischung oder ein Material aus, das durch Kompression von einer ersten, geringeren Dichte bzw. einem ersten geringeren spezifischen Gewicht auf eine zweite, höhere Dichte oder ein höheres spezifisches Gewicht überführbar ist. Dabei können die Hohlräume geschlossen und mit einem Gas wie beispielsweise Luft oder Stickstoff oder einem Edelgas bzw. einem anderen bioinerten Gas gefüllt sein, das durch Druck leicht volumenkomprimierbar ist.

Derartige geschlossene Hohlräume tendieren dazu, sich bei Wegfall einer äußeren Druckkraft durch die Gaselastizität wieder auszudehnen, so dass das Gehäuse sich, sobald er an den Einsatzort gebracht ist, selbsttätig wieder entfalten kann. Zumindest aber wird die Entfaltungsbewegung durch die Gaselastizität unterstützt.

Es können zudem allerdings auch Gasleitungen zu dem Gehäuse vorgesehen sein, die in einem oder mehreren Hohlräumen enden und das Aufpumpen der Hohlräume aktiv erlauben. Über die gleichen Leitungen kann gegebenenfalls auch das Gas zur Komprimierung abgesaugt werden.

Ebenso kann mit einer Flüssigkeit verfahren werden, wenn diese in die Hohlräume eingeführt wird. Befindet sich eine Flüssigkeit in den Hohlräumen, so ist diese üblicherweise sehr viel weniger komprimierbar, jedoch kann sie durch die geeignete Wahl der Viskosität im Zusammenspiel mit den übrigen konstruktiven Teilen des Gehäuses eine hohe Beweglichkeit und damit Komprimierbarkeit ermöglichen und dennoch im Betrieb durch die Inkompressibilität nach Entfaltung des Gehäuses eine gewisse Gehäusestabilität unterstützen.

Die Hohlräume können auch offen gestaltet werden, wobei damit ebenfalls eine hohe Kompressibilität gegeben ist. Das Material, das die Hohlräume begrenzt, muss dann entsprechend elastisch ausgebildet sein. Dies kann zum Beispiel bei einem offenporigen Schaumstoff gegeben sein.

Die Erfindung kann auch vorteilhaft dadurch ausgeführt werden, dass der/die Hohlraum/Hohlräume wenigstens teilweise von einer teildurchlässigen Membran begrenzt ist/sind.

In diesem Fall kann ein Hohlraum mit einer Flüssigkeit gefüllt sein, die zusammen mit der eingesetzten Membran und in Abhängigkeit von der Flüssigkeit, in der die Pumpe einsetzbar ist, insbesondere menschlichem Blut, aufgrund von Osmose eine Diffusion in den Hohlraum hinein zulässt, die zu einer Druckerhöhung und zu einem Aufpumpen des Gehäuses führt.

Ebenso können auch Stoffe eingesetzt werden, die nach dem Inkontakttreten mit der zu fördernden Flüssigkeit zu Schwellprozessen infolge von Aufsaugen der Flüssigkeit führen und somit über eine Volumenzunahme eine Dekomprimierung des Gehäuses unterstützen.

Im Falle des Osmoseprozesses bietet sich das Füllen der Hohlräume mit einem Salz oder einer Salzlösung an, deren Salzkonzentration höher ist als die der zu fördernden Flüssigkeiten.

Vorteilhaft kann auch vorgesehen sein, dass zumindest der überwiegende Teil der Hohlräume von festem Material des Gehäuses umgeben und über Öffnungen mit dem Außenbereich und/oder untereinander verbunden ist/sind. In diesem Fall kann bei der Kompression ein Fluidtransport über die Hohlräume und gegebenenfalls auch aus dem Gehäuse heraus geschehen, so dass die entsprechenden Hohlräume leicht vollständig komprimierbar sind.

Das Gehäuse kann beispielsweise teilweise aus einem porösen Material wie Schaumstoff, insbesondere Polyurethan, bestehen. Ein solcher Schaumstoff kann offen- oder geschlossenporig sein. Im Falle eines offenporigen Schaumstoffs beruht die Elastizität auf dem stützenden, die Poren umgebenden Material, das sich nach einer Komprimierung von selbst wieder in seine ursprüngliche Form bewegt, wobei das Gas oder Fluid in die Poren zurückströmen kann. Durch die begrenzten Strömungsquerschnitte der Verbindungen der Hohlräume/Poren untereinander ist eine Zeitkonstante bei der Komprimierung/Dekomprimierung in gewissen Grenzen wählbar. Diese kann dafür sorgen, dass im Betrieb der Pumpe schlagartigen Deformationen des Gehäuses durch ungleichmäßige mechanische Belastung entgegengewirkt wird.

Es kann vorgesehen sein, ein derartiges Gehäuse durch Spritzen eines Schaumstoffs in eine vorgefertigte Form zu erzeugen.

Im Folgenden wird die Erfindung anhand eines Ausführungsbeispiels in einer Zeichnung gezeigt und anschließend beschrieben.

Dabei zeigt
- Fig. 1: eine Übersicht über den Einsatz einer erfindungsgemäßen Fluidpumpe in einem Blut- gefäß,
- Fig. 2: einen Längsschnitt durch eine Fluidpumpe bei komprimiertem Förderelement,
- Fig. 3: einen Längsschnitt wie in Fig. 2 mit ex- pandiertem Förderelement,
- Fig. 4: einen Längsschnitt durch ein Gehäuse mit Stabilisierungskammern,
- Fig. 5: einen Querschnitt durch die Konstruktion gemäß Fig. 4,
- Fig. 6: einen Längsschnitt durch ein Gehäuse mit einer weiteren Gestaltung von Stabilisie- rungskammern,
- Fig. 7: einen Querschnitt wie in Fig. 6 angezeigt,
- Fig. 8: einen Längsschnitt durch ein hohlballonar- tiges Gehäuse in rotationselliptischer Form,
- Fig. 9: einen Längsschnitt durch das Funktions- element insgesamt in komprimierter Form,
- Fig. 10: eine Fluidpumpe im Längsschnitt mit einem Pumpenrotor,
- Fig. 11: in einer Seitenansicht einen nabenlosen Rotor und
- Fig. 12: eine Detailansicht einer Porenstruktur.

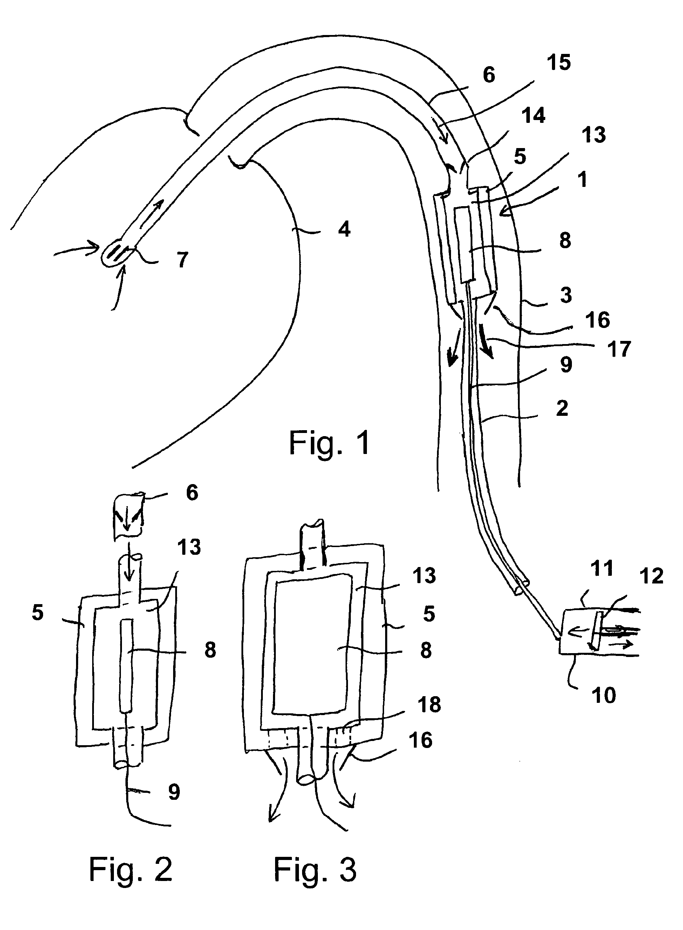

Fig. 1 zeigt das Funktionselement 1 in Form einer Fluidpumpe in einem Schnitt, eingesetzt im menschlichen Körper. Die Fluidpumpe ist mittels eines Hohlkatheters 2 in ein Blutgefäß 3 eingeführt, das zu einer Herzkammer 4 führt. Das Gehäuse 5 der Fluidpumpe ist über einen Ansaugschlauch 6, der durch das Blutgefäß 3 verläuft, mit dem Inneren der Herzkammer 4 verbunden und der Ansaugschlauch weist dort eine oder mehrere Ansaugöffnungen 7 auf. Der Ansaugschlauch 6 ist an seinem Ende in der Nähe der Ansaugöffnungen 7 abgerundet, um Verletzungen des Herzinnenbereichs zu vermeiden.

Innerhalb des Gehäuses 5 der Fluidpumpe befindet sich ein Förderelement 8 in Form eines Ballons, im vorliegenden Fall im Wesentlichen in zylindrischer Form, der über eine Druckleitung 9 mit einer Druckquelle 10 außerhalb des Körpers verbunden ist. Die Druckleitung 9 verläuft durch den Hohlkatheter 2, und beide werden in einer nicht dargestellten Schleuse aus dem Blutgefäß 3 zum Körperäußeren geführt.

Die Druckquelle 10 ist nur schematisch in Form eines Zylinders 11 und eines darin beweglichen Kolbens 12 gezeigt, wobei der Kolben bei pulsierender Bewegung einen ebenfalls pulsierenden Druck erzeugt, der zu einer abwechselnden Expansion und Kompression des Förderelements 8 führt.

In der Folge nimmt das Förderelement 8 im Inneren des Gehäuses 5 wechselweise mehr oder weniger Raum ein, so dass im Gegenzug mehr oder weniger Raum für das zu fördernde Fluid im Fluidraum 13 des Gehäuses 5 übrig bleibt. Das Fluid wird somit durch das Förderelement 8 pulsierend verdrängt und angesaugt.

Durch den Einsatz von Ventilklappen wird der Fluidstrom in gewünschter Form gerichtet. In dem dargestellten Beispiel sind Ventilklappen 14 in der Ansaugleitung 6 vorgesehen, die das Fluid in Richtung des Pfeils 15 einströmen lassen. Die Ventilklappen könnten ebenso gut in der entsprechenden Öffnung des Gehäuses 5 vorgesehen sein. Es handelt sich dabei um ein Rückschlag- bzw. Einwegventil, das das Fluid in Richtung des Pfeils 15 einströmen, jedoch nicht in entgegengesetzter Richtung ausströmen lässt. Dies führt dazu, dass bei Komprimierung des Förderelements 8 über diesen Weg Fluid, also insbesondere Blut, angesaugt werden kann, dass dies jedoch bei Expansion des Förderelements dort nicht wieder ausströmen kann.

Zum Ausströmen sind Ausströmklappen 16 vorgesehen, die beispielsweise direkt am Gehäuse 5 angeordnet sein können und die ebenfalls das Fluid nur in einer Richtung, nämlich in Richtung der Pfeile 17, ausströmen lasen.

Im Zusammenwirken der Ventilklappen 14, 16 ist gewährleistet, dass das Fluid nur in Richtung der Pfeile 15, 17 von der Fluidpumpe gefördert wird.

Der Durchsatz der Fluidpumpe wird außer von der Restleistung des Patientenherzens, soweit dies noch funktioniert, von der Pulsierfrequenz des Förderelements 8 einerseits und von dem durch das Förderelement jeweils verdrängten Volumen bzw. dem in dem Fluidraum 13 verbleibenden freien Volumen bestimmt. Maximiert ist die Förderung dann, wenn das Förderelement sich bis zum völligen Ausfüllen des Fluidraums 13 ausdehnen kann und danach so weit zusammenfällt, dass sein Innenraum völlig geleert ist. Das Förderelement kann zu diesem Zweck aus einem hochelastischen Werkstoff bestehen, der nach Absenkung der Drucks in der Druckleitung 9 für eine Komprimierung des Förderelements sorgt. Dies führt zu einer Druckabsenkung im Fluidraum 13, wodurch weiteres Fluid nachgesaugt wird.

Voraussetzung für das Funktionieren dieses Mechanismus ist allerdings, dass das Gehäuse 5 stabil bleibt und auch bei Erzeugen eines Unterdrucks in seinem Inneren nicht zusammenfällt. Diese Anforderung ist gemäß der Erfindung mit der weiteren Anforderung verbunden, dass das Gehäuse zum Einbringen und zur Entfernung in und aus einem Körper komprimierbar sein muss.

Die Erfindung sieht dazu vor, das Gehäuse mit wenigstens einer Stabilisierungskammer zu versehen, die durch Druckbeaufschlagung das Gehäuse versteift.

Dabei kann in einem Extremfall das gesamte Gehäuse als doppelwandiger Ballon ausgebildet sein, wobei im expandierten Zustand der Raum zwischen den Ballonwänden typischerweise unter höherem Druck steht als der das Gehäuse umgebenden Raums des Blutgefäßes. Es kann auch vorgesehen sein, dass dieser Druck höher ist als der Druck, der maximal im Förderelement und in dem Fluidraum herrscht.

Fig. 2 zeigt schematisch den Zustand einer Fluidpumpe mit einem hohlzylinderförmigen Gehäuse 5 und einem komprimierten Förderelement 8 in der Ansaugphase. Der Fluidraum 13 wird im Wesentlichen mit dem durch die Ansaugleitung 6 strömenden Fluid gefüllt.

Das Förderelement 8 weist eine im Wesentlichen zylindrische Form auf und besteht beispielsweise aus Gummi oder Polyurethan oder einem Elastomer mit ähnlichen Eigenschaften, wobei die Oberfläche des Förderelements mit einem Stoff beschichtet sein kann, der einerseits Infektionen verhindert, andererseits die Anlagerung von Blut an der Oberfläche vermeidet. Eine ebensolche Beschichtung kann im Innenraum des Gehäuses 5 an dessen Wänden vorgesehen sein.

Das Förderelement 8 ist über eine Druckleitung 9, die im Wesentlichen nicht expandierbar gestaltet ist, mit einer Druckquelle verbunden.

Fig. 3 zeigt die Konfiguration aus Fig. 2 in einem expandierten Zustand des Förderelementes 8, in dem der freie Restraum des Fluidraums 13 minimiert ist und das Fluid / das Blut durch Ventilklappen 16 aus Öffnungen 18 des Gehäuses 5 ausströmen kann.

Es ist zu bemerken, dass das Gehäuse 5 bezüglich seines Außendurchmessers so gestaltet sein kann, dass es die lichte Weite des Blutgefäßes 3 nicht vollständig ausfüllt, so dass beim Einsatz in einen Körper durch die Eigenfunktion des Herzens Blut neben der Fluidpumpe her durch das Gefäß 3 gefördert werden kann.Es ist allerdings auch denkbar, dass der Durchmesser des Blutgefäßes durch einen geeignet gewählten Durchmesser auch zu bestimmten Zwecken voll ausgefüllt wird.

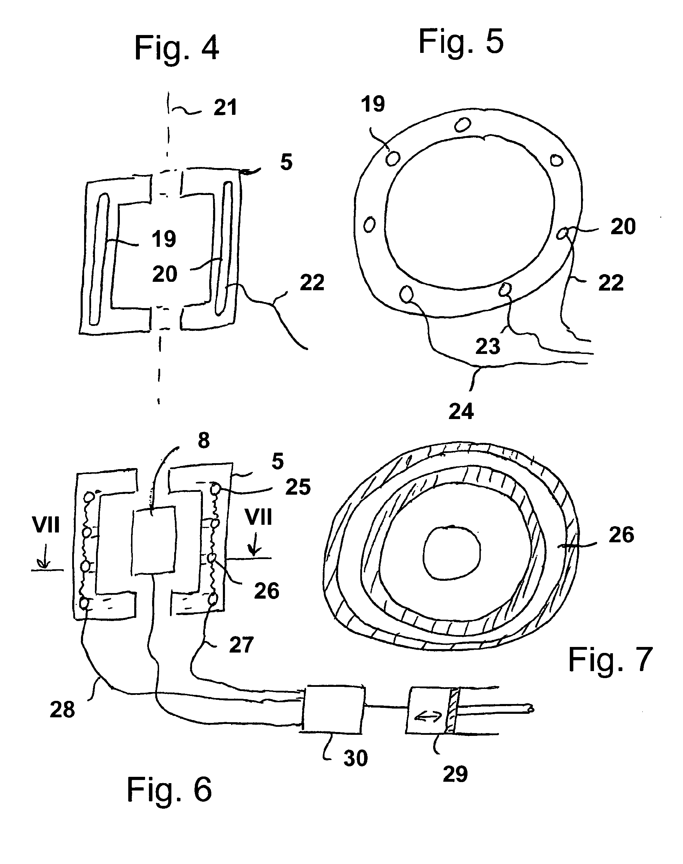

In Fig. 4 ist genauer auf die Verstärkung des Gehäuses 5 durch Stabilisierungskammern eingegangen. In diesem Ausführungsbeispiel sind mehrere strangförmige Stabilisierungskammern 19, 20 parallel zur Längsachse 21 des Gehäuses 5 ausgerichtet und in der Gehäusewand angeordnet.

Fig. 5 zeigt einen Querschnitt mit sieben solcher Stabilisierungskammern. Die einzelnen Stabilisierungskammern sind in diesem Fall voneinander getrennt und einzeln über Druckleitungen 22, 23, 24 mit einer Fluiddruckquelle verbunden. Die Stabilisierungskammern können druckbeaufschlagt werden, um das Gehäuse 5 zu versteifen. Zum Einbringen bzw. Entfernen des Gehäuses 5 werden sie geleert, so dass das Gehäuse 5 in sich zusammenfallen kann.

Die Stabilisierungskammern können auch untereinander über eine Druckleitung miteinander verbunden sein, um zu gewährleisten, dass in ihnen jeweils gleicher Druck herrscht, und um das Befüllen und Entleeren zu vereinfachen.

Fig. 6 zeigt eine andere Anordnung der Stabilisierungskammern in Form von ringförmigen Strängen, die jeweils koaxial zueinander und zu dem Gehäuse 5 angeordnet sind. Die Stabilisierungskammern sind mit 25, 26 bezeichnet. In dem Ausführungsbeispiel sind vier dieser Stabilisierungskammern äquidistant zueinander in der Gehäusewand vorgesehen. Sie sind mittels Druckleitungen 27, 28 mit einer Druckquelle 29 verbunden.

Derartige ringförmige oder auch denkbare, spiralförmige Stabilisierungskammern bieten eine besonders gute Versteifung des Gehäuses und bei Druckbeaufschlagung einen entsprechenden Widerstand gegen den pulsierenden Unterdruck im Gehäuseinneren.

In dem Ausführungsbeispiel sind die Stabilisierungskammern und das Förderelement 8 mit derselben Druckquelle 29 über ein Mehrwegeschaltventil 30 verbunden. Dadurch kann die Druckquelle 29 sowohl zum Befüllen der Stabilisierungskammern als auch zum pulsierenden Pumpen des Förderelements 8 verwendet werden.

Fig. 7 zeigt einen Querschnitt durch das in Fig. 6 im Längsschnitt dargestellte Gehäuse mit einer torusförmigen Stabilisierungskammer 26.

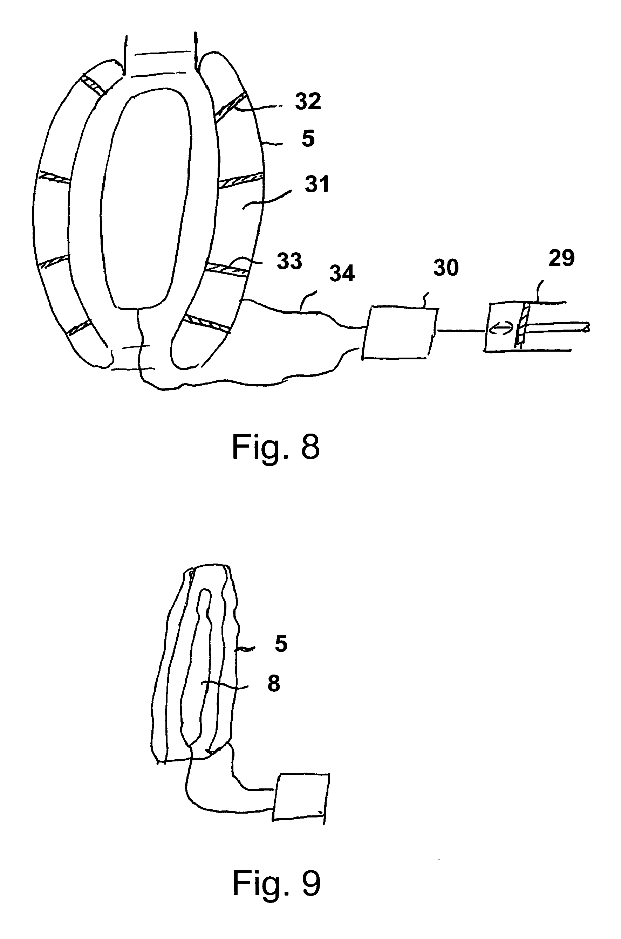

In Fig. 8 ist ein anderes Konzept des Gehäuses im Rahmen der Erfindung dargestellt, bei dem die Gehäusewand doppelwandig, die beiden Außenwände(Ballonwände)sehr dünn gestaltet und das Gehäuse damit ballonartig aufgebaut ist. Das Gehäuse bildet einen doppelwandigen Ballon mit einem großen Hohlraum 31, der jedoch in mehrere Teilräume unterteilt sein kann. Entweder sind zu diesem Zweck dünne Zwischenwände vorgesehen, oder, wenn keine vollständige Unterteilung gewünscht ist, können auch nur einzelne Verstärkungsstreben in Form von Stegen 32, 33 vorgesehen sein. Diese sorgen dafür, dass Innen- und Außenwand des Ballons keine Scherbewegungen gegeneinander ausführen können, so dass das Gehäuse 5 insgesamt stabilisiert wird. Der Innenraum 31 ist mittels einer Druckleitung 34 mit der Druckquelle 29 verbunden. Das Gehäuse 5 weist insgesamt die Kontur eines Rotationsellipsoids auf.

Die Stabilisierungsstreben 32, 33 können typischerweise aus demselben Material bestehen wie die Ballonwände des Gehäuses 5 und einstückig mit diesem hergestellt sein. Die Streben können dabei ringförmig um das Gehäuse 5 umlaufen oder als achsparallele Stege ausgebildet sein. Es ist jedoch auch jede Ausrichtung, beispielsweise auch eine gitterförmige Struktur, denkbar.

Ebenso ist auch in den oben beschriebenen Ausführungsformen nicht nur die konkret beschriebene Ausrichtung der strangförmigen Stabilisierungskammern denkbar, sondern auch dort eine gitterförmige bzw. netzförmige Struktur, die eine massive Gehäusewand durchsetzt.

In der Fig. 9 ist schließlich die kollabierte Form des in der Fig. 8 dargestellten Gehäuses gezeigt, wobei das Rotationsellipsoid zusammengefallen ist. Das Förderelement 8 ist selbstverständlich zum Einbringen/Ausbringen der Fluidpumpe in ein Blutgefäß ebenfalls zu komprimieren.

Zum Entnehmen der Fluidpumpe aus dem Gefäß kann vorgesehen sein, dass zunächst das Gehäuse 5 durch Druckabsenkung in den Stabilisierungskammern kollabiert wird und erst danach das Förderelement 8 entleert wird. Dadurch ergibt sich beim Zusammenfalten des Gehäuses 5 eine längliche Form, und es wird verhindert, dass dieses vorwiegend in Längsrichtung kollabiert und damit einen höheren Querschnitt einnimmt. Auch kann so das elastische Kontrahieren des Gehäuses das Komprimieren des Förderelementes unterstützen.

Durch die Erfindung wird ein in hohem Maße komprimierbares Funktionselement gebildet, das dennoch im Betrieb die notwendige Stabilität aufweist, um sowohl Unter- als Überdrücken beim pulsierenden Pumpen formstabil zu widerstehen.

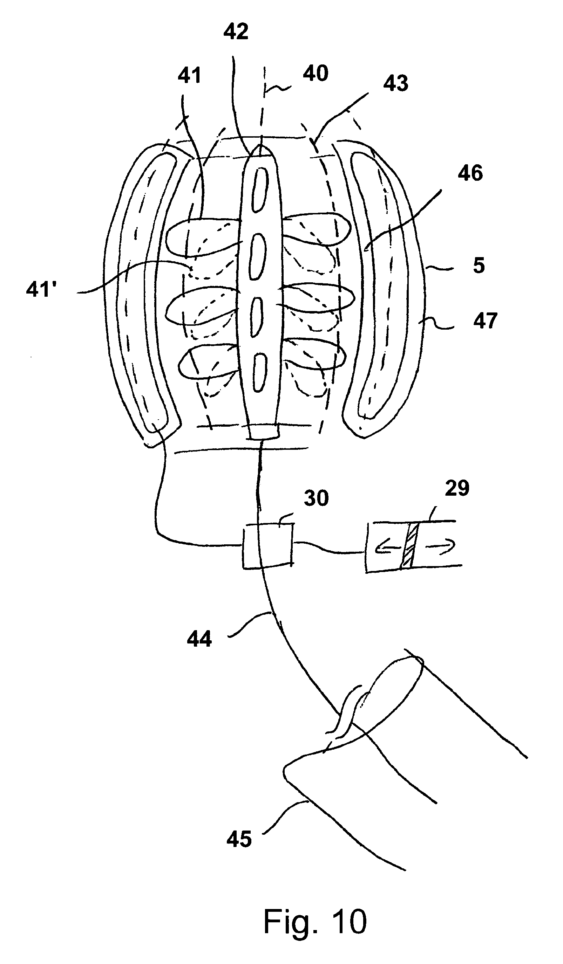

In Figur 10 ist ein Gehäuse 5 ähnlich dem in Figur 9 dargestellten gezeigt, dass hier allerdings einen Pumpenrotor umgibt, der um seine Längsachse 40 rotiert und dabei axial ein Fluid fördert. Die Schaufelblätter 41 können dabei einzeln an einem Schaft 42 abbiegbar befestigt sein oder es kann auch ein wendelförmig umlaufendes Schaufelblatt vorgesehen sein. Der Rotor ist radial komprimierbar und kann beispielsweise aus einem Gerüst aus einer Gedächtnislegierung, beispielsweise Nitinol, gefertigt sein, oder aus einem anderen elastischen Werkstoff. Der Rotor kann in den Schaufelblättern und/der der Nabe offene oder geschlossene Hohlräume aufweisen, die elastisch komprimierbar sind.
In Figur 10 ist die Innenwand des Gehäuses mit 46, die Außenwand mit 47 bezeichnet. Wenigstens die Innenwand ist elastisch aufweitbar und kontrahierbar. Gestrichelt ist ein kontrahierter Zustand des Gehäuses 5 dargestellt und die Innenwand ist mit 43 bezeichnet. Diese drückt in dem dargestellten Zustand die Schaufelblätter 41' in einen radial eingebogenen Zustand, so dass der gesamte Rotor bereits ein Stück weit radial verkleinert ist.

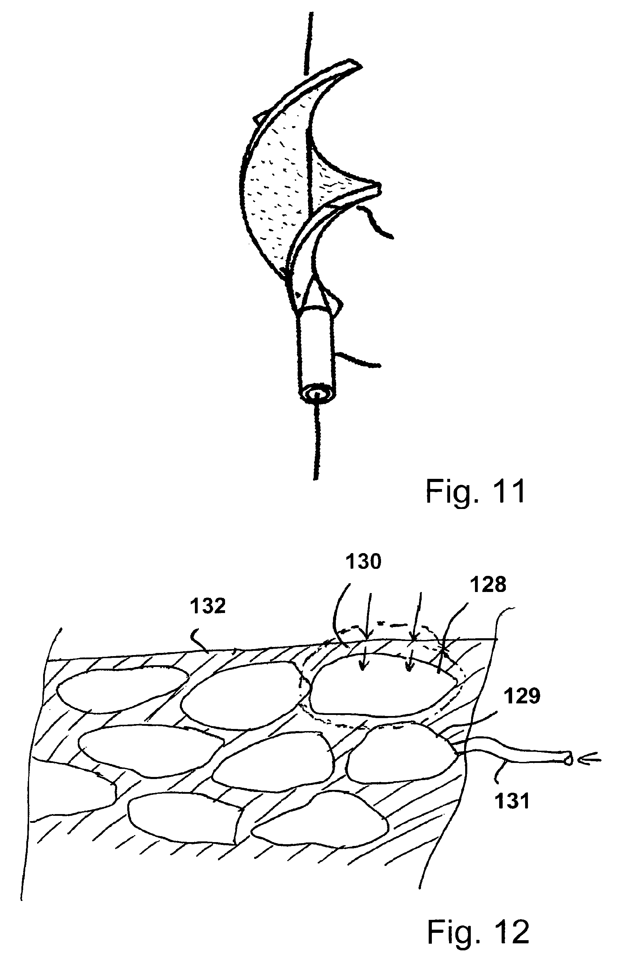

In Figur 11 ist ein ebenso verwendbarer, nabenloser Rotor dargestellt, der aus einer spiralförmig gebogenen flachen Platte besteht. Diese kann zum Beispiel aus einem leicht verformbaren oder komprimierbaren Stoff bestehen, wie beispielsweise einem mit Folie bespannten Blechgitter oder einem Schaumstoff, der offenporig oder geschlossenporig sein kann.
Der dargestellte Rotor ist nabenlos und selbsttragend, das heißt, das Schaufelblatt überträgt selbst das Drehmoment und ist nur außerhalb drehbar gelagert und mit einer Antriebswelle 44 verbunden (Fig. 10), die durch einen Hohlkatheter 45 verläuft.

Fig. 12 zeigt in stark vergrößerter, mikroskopischer Darstellung ein Gehäusematerial in Form eines Schaumstoffs 132 mit geschlossenen Poren 128, 129, wobei in einer Variante (Hohlraum 128) das Material der Wände zwischen den Poren als semipermeable Membran ausgebildet ist.

Eine solche Membran lässt die Diffusion bestimmter Flüssigkeiten zu, was beispielsweise für einen osmotischen Effekt genutzt werden kann. Werden die Hohlräume/Poren 128 beispielsweise mit einer Flüssigkeit gefüllt, in der hochkonzentriert ein Salz gelöst ist, und wird der Schaumstoff in eine Flüssigkeit gebracht, die eine geringere Lösungskonzentration aufweist, so tendiert die Konstellation dazu, die Konzentrationen beider Flüssigkeiten dadurch aneinander anzunähern, dass das Lösungsmittel von außen in das Innere das Hohlraums 128 durch die Membran 130 eindiffundiert. Hierdurch wird ein erhöhter osmotischer Druck erzeugt, der zum Aufpumpen des Hohlraums 128 bis in die gestrichelt dargestellte Form genutzt werden kann. Hierdurch kann eine Expansion und Versteifung des Schaumstoffs realisiert werden.

Dieser Effekt kann auch für größere Hohlräume im Gehäuse gezielt genutzt werden. Alternativ können auch Quellvorgänge zur Expansion des Rotors genutzt werden.

Im Zusammenhang mit dem Hohlraum 129 ist ein Schlauch 131 dargestellt, der symbolisiert, dass entsprechende Hohlräume auch über einzelne oder kollektive Zuleitungen mit einem Fluid gefüllt werden können oder dass ein solches Fluid aus ihnen abgesaugt werden kann, um entsprechende Dekompressions-/Kompressionsvorgänge zu steuern.

## Patentansprüche

1. Funktionselement (1), insbesondere Fluidpumpe, mit einem einen Fluidraum (13) begrenzenden Gehäuse (5) und mit einem in dem Fluidraum angeordneten Interaktionselement (8), insbesondere Förderelement für das Fluid, wobei sowohl das Gehäuse als auch das Interaktionselement bezüglich seiner Form und/oder Größe zwischen wenigstens einem ersten, expandierten und einem zweiten, komprimierten Zustand veränderbar ist, wobei das Gehäuse wenigstens eine mit einem Fluiddruck beaufschlagbare, von dem Fluidraum verschiedene Stabilisierungskammer (19,20,25,26,31) zwischen einer Innenwand (46) und einer Außenwand (47) des Gehäuses aufweist, wobei einem ersten Fluiddruck in der Stabilisierungskammer der erste Zustand des Gehäuses und einem zweiten Fluiddruck der zweite Zustand des Gehäuses zugeordnet ist und wobei die Innen- und/oder Außenwand des Gehäuses elastisch ist, und sich beim Übergang vom ersten in den zweiten Zustand elastisch zusammenzieht.

2. Funktionselement, insbesondere Fluidpumpe, nach Anspruch 1, **dadurch gekennzeichnet, dass** im zweiten Zustand der Durchmesser des Gehäuses durch die Eigenelastizität soweit reduziert ist. Dass das Interaktionselement in seinem expandierten Zustand durch die Innenwand radial zusammengedrückt wird.

3. Funktionselement, insbesondere Fluidpumpe, nach Anspruch 1 oder 2, **dadurch gekennzeichnet, dass** dem ersten, expandierten Zustand des Gehäuses (5) ein erster, höherer Fluiddruck in der Stabilisierungskammer (19,20,25,26,31) zugeordnet ist als dem zweiten, komprimierten Zustand des Gehäuses.

4. Funktionselement, insbesondere Fluidpumpe, nach Anspruch 1,2 oder 3, **dadurch gekennzeichnet, dass** das Gehäuse (5) wenigstens abschnittsweise die Form eines Hohlzylinders, eines Torus oder eines Hohlrotationsellipsoids aufweist.

5. Funktionselement insbesondere Fluidpumpe nach Anspruch 1, 2,3 oder 4, **dadurch gekennzeichnet, dass** wenigstens eine Stabilisierungskammer (19,20,25,26,31) in Form eines in dem expandierten Gehäusezustand strangförmigen Hohlraums, insbesondere innerhalb einer Gehäusewand, vorgesehen ist.

6. Funktionselement, insbesondere Fluidpumpe, nach Anspruch 5, **dadurch gekennzeichnet, dass** wenigstens drei Stabilisierungskammern (19,20,25,26) in Form von im ersten Gehäusezustand strangförmigen Hohlkammern vorgesehen sind.

7. Funktionselement, insbesondere Fluidpumpe, nach Anspruch 4 und 5, **dadurch gekennzeichnet, dass** der wenigstens eine strangförmige Hohlraum (25,26) in Umfangsrichtung des Hohlzylinders, Torus oder Hohlrotationsellipsoids ringförmig oder wendelförmig umlaufend ausgebildet ist.

8. Funktionselement, insbesondere Fluidpumpe, nach Anspruch 5,6 oder 7, **dadurch gekennzeichnet, dass** wenigstens eine Stabilisierungskammer (19,20) parallel zur Längsachse des Hohlzylinders, Torus oder Hohlrotationsellipsoids in dessen Wandung verläuft.

9. Funktionselement, insbesondere Fluidpumpe, nach Anspruch 1, 2,3 oder 4, **dadurch gekennzeichnet, dass** die Stabilisierungskammer (19,20,25,26,31) den Raum der Gehäusewand im Wesentlichen ausfüllt.

10. Funktionselement, insbesondere Fluidpumpe, nach einem der vorangehenden Patentansprüche, **dadurch gekennzeichnet, dass** die Stabilisierungskammer/n wenigstens teilweise von jeweils wenigstens einem Steg (32,33) durchsetzt sind.

11. Funktionselement, insbesondere Fluidpumpe, nach einem der vorangehenden Patentansprüche, **dadurch gekennzeichnet, dass** das Gehäuse (5) die Bauform eines hohlwandigen Ballons aufweist.

12. Funktionselement, insbesondere Fluidpumpe, nach einem der vorangehenden Patentansprüche, **dadurch gekennzeichnet, dass** das Gehäuse (5) eine Einströmöffnung und eine Ausströmöffnung aufweist, die jeweils mit einem Ventil (14,16), insbesondere einem Einwegeventil versehen sind.

13. Fluidpumpe nach einem der vorangehenden Patentansprüche, **dadurch gekennzeichnet, dass** das Förderelement (8) als radial komprimierbarer Rotor mit wenigstens einem Schaufelblatt ausgebildet ist.

14. Fluidpumpe nach Anspruch 13, **dadurch gekennzeichnet, dass** das/die Schaufelblatt/Schaufelblätter elastisch radial zur Rotorachse hin biegbar ist/sind.

15. Fluidpumpe nach Anspruch 13 oder 14, **dadurch gekennzeichnet, dass** der Rotor wenigstens teilweise aus einem volumenkompressiblen Material, insbesondere einem Schaumstoff, besteht.

16. Fluidpumpe nach Anspruch 13, 14 oder 15, **dadurch gekennzeichnet, dass** der Rotor komprimierbare Hohlräume aufweist.

17. Fluidpumpe nach Anspruch 13, 14, 15 oder 16,
**dadurch gekennzeichnet, dass** der Rotor nabenlos gestaltet ist.

18. Fluidpumpe nach einem der vorangehenden Patentansprüche, **dadurch gekennzeichnet, dass** das Förderelement (8) als
wenigstens ein fluidfüllbarer Ballon ausgeführt ist.

19. Fluidpumpe nach Anspruch 11, **dadurch gekennzeichnet, dass** das Förderelement (8) in gefüllter Form den Fluidraum des Gehäuses im Wesentlichen ausfüllt.

20. Fluidpumpe nach Anspruch 12 oder 13, **dadurch gekennzeichnet, dass** das Förderelement (8) mit demselben Fluid gefüllt ist wie die Stabilisierungskammer/n.

21. Fluidpumpe nach Anspruch 12, 13 oder 14, **dadurch gekennzeichnet, dass** das Förderelement (8) und die Stabilisisierungskammern des Gehäuses (5) mit derselben Fluiddruckquelle verbindbar sind.

22. Funktionselement (1), insbesondere Fluidpumpe, mit einem einen Fluidraum (13) begrenzenden Gehäuse (5) und mit einem in dem Fluidraum angeordneten Interaktionselement (8), insbesondere Förderelement für das Fluid, wobei sowohl das Gehäuse als auch das Interaktionselement bezüglich seiner Form und/oder Größe zwischen wenigstens einem ersten, expandierten und einem zweiten, komprimierten Zustand veränderbar ist, wobei die mittlere Dichteänderung des Gehäusematerials zwischen dem ersten und zweiten Zustand mindestens 10 % beträgt.
